# EUROPEAN PATENT SPECIFICATION

(11) **EP 1 518 554 B1**
(45) Date of publication and mention of the grant of the patent: **14.05.2008**
(21) Application number: 04104599.8
(22) Date of filing: 22.09.2004
(51) Int. Cl.: A61K 31/205, A61K 31/519, A61K 31/714, A61P 3/00

(54) **Pharmaceutical composition for the treatment of hyperhomocysteinemia**
Pharmazeutische Zusammensetzung zur Behandlung von Hyperhomocysteinämie
Composition pharmaceutique pour le traitement de l'hyperhomocystéinémie

(30) Priority: 26.09.2003 IT RM20030442
(43) Date of publication of application: 30.03.2005
(73) Proprietor: Medosan Industrie Biochimiche Riunite S.r.l., 00040 Cecchina di Albano Laziale RM (IT)
(72) Inventor: Anzalone, Sergio Medosan Ind. Biochim. Riunite srl, 00040 Cecchina di Albano Laziale RM (IT)
(74) Representative: Bazzichelli, Alfredo

(56) References cited:
- WO-A-01/56609
- WO-A-03/020260
- WO-A-03/068231
- US-A- 6 054 128
- DATABASE MEDLINE [Online] US NATIONAL LIBRARY OF MEDICINE (NLM), BETHESDA, MD, US; January 2004 (2004-01), DI SABATO F: "[Comparison of treatment with STRESSEN and arginine glutamate on homocysteine blood levels]" XP002312740 Database accession no. NLM15147076 & LA CLINICA TERAPEUTICA. JAN 2004, vol. 155, no. 1, January 2004 (2004-01), pages 17-20, ISSN: 0009-9074
- DATABASE MEDLINE [Online] US NATIONAL LIBRARY OF MEDICINE (NLM), BETHESDA, MD, US; February 2004 (2004-02), MONTELEONE F: "[Study of the effect of Stressen on homocysteine blood levels in patients affected by vascular disease]" XP002312741 Database accession no. NLM15244107 & LA CLINICA TERAPEUTICA. 2004 FEB-MAR, vol. 155, no. 2-3, February 2004 (2004-02), pages 57-59, ISSN: 0009-9074
- DATABASE MEDLINE [Online] US NATIONAL LIBRARY OF MEDICINE (NLM), BETHESDA, MD, US; February 2004 (2004-02), MONTELEONE F: "[Controlled study of the effect of Stressen on homocysteine blood levels in patients with vascular disease]" XP002312742 Database accession no. NLM15244109 & LA CLINICA TERAPEUTICA. 2004 FEB-MAR, vol. 155, no. 2-3, February 2004 (2004-02), pages 65-67, ISSN: 0009-9074

## Description

The present invention relates to the use of folic acid, vitamin B₁₂ and arginine glutamate for the production of a medicament for the treatment of hyperhomocysteinemia and a pharmaceutical composition to be used to such purpose.

The hyperhomocysteinemia, as suggested by the epidemiological clinic evidence, is associated to an increased risk of cardiovascular and thromboembolic diseases; in patients with acute coronary syndrome it has been noted that elevated plasmatic levels of homocysteine are highly predictive of long term prognoses of cardiac events.

Furthermore, a consistent scientific literature, related to the risk factors for cardiovascular pathologies, underlines a strict, inversely proportional correlation between the ematic levels of homocysteine and the ones of folates and vitamin B₁₂, which are involved in the metabolism of such aminoacid.

It is known from the scientific literature that administrations of folic acid and vitamin B₁₂ lower the homocysteine plasmatic levels.

Clinic data, in fact, demonstrate that a therapy with folic acid (0.5-5 mg a day) and vitamin B₁₂ (0.5-1 mg a day) reduces the homocysteine plasmatic levels by 25-30%, thereby decreasing the cardiovascular risk.

Nevertheless, it is also known from the scientific literature that quantities of folic acid and vitamin B₁₂ greater than the ones just indicated do not produce additional lowerings in the homocysteine plasmatic levels. British Medical Journal, Vol 316, March, 21st 1998 includes the article "Lowering blood homocysteine with folic acid based supplements: meta-analysis of randomised trials" wherein, for example, it is stated that to an increase in the quantity of administered folic acid (in the range 0.5-5 mg a day) does not correspond a respective lowering in the homocysteine plasmatic concentration. Therefore, the state of art did not include any indication to increase the administered concentration of folic acid in order to have an additional lowering in the homocysteine plasmatic concentration.

Folates can be taken by oral route. The mechanism with which folates and vitamin B₁₂ determine the reduction in the homocysteine plasmatic content is based upon the use of the methyl group of the methyltetrahydrofolate by the cobalamin, with consequent formation of methylcobalamin; this molecule allows converting homocysteine into methionine by transferring a methyl group.

The role played by elevated plasmatic levels of homocysteine in the pathogenesis of cardiovascular and thromboembolic events can be explained by the action modes of the molecule itself: in fact, homocysteine can induce the activation of the blood platelets and of the coagulation system, thereby also causing the reduction in the NO bioavailability; a secondary endothelial dysfunction with activation of endothelial cells and monocytes, by exciting inflammatory cytokines which induce a progressive atherosclerosis results therefrom. Such functional alterations of the vascular endothelium intervene in the pathogenesis and in the clinical phenomena of the cardiovasculopathies.

Notwithstanding the good results obtained with the administration of folic acid and vitamin B₁₂, however, in the state of art it was felt the need to make available products, or pharmaceutical compositions, with increased effectiveness in the treatment of hyperhomocysteinemia and, at the same time, to proceed with an administration of substances able to guarantee a safe and long-term administration.

From the state of art a pharmaceutical formulation is known, authorized to be marketed in Italy, containing folinic acid in the form of calcium pentahydrate salt in quantity of 5-mg, cobamamide in quantity of 1-mg and arginine glutamate in quantity of 1000-mg. Nevertheless, this formulation is used and authorized for treating psychophysical stress.

It has now surprisingly been found that the administration of arginine glutamate in addition to folic acid and vitamin B₁₂ allows obtaining an additional reduction in the homocysteine plasmatic content, by increasing the action of folic acid and vitamin B₁₂. Due to the administration also of arginine glutamate the reduction in the homocysteine plasmatic levels goes from 25 - 30 % to 50-55 %.

Therefore, the object of the present invention is the use of folic acid, vitamin B₁₂ and arginine glutamate for the production of a medicament for the treatment of hyperhomocysteinemia. In particular, in said medicament the folic acid is present in the form of folinic acid and the vitamin B₁₂ in the form of cobamamide. In said medicament, the folinic acid, preferably in the form of calcium pentahydrate salt, is contained in quantities from 0.1 mg to 25 mg, the cobabamide between 0.1 to 5 mg, the arginine glutamate in a quantity between 0.1 to 5000 mg.

Furthermore, it has surprisingly been found, and this too is an object of the present invention, that the administration of arginine glutamate alone reduces the homocysteine plasmatic levels and therefore the same compound can be used for the treatment of hyperhomocysteinemia. Experimental data obtained by the Applicant of the present application demonstrate that, after administration of arginine glutamate, the reduction in the homocysteine plasmatic levels is 10-15%.

Therefore, an additional object of the present invention is the use of arginine glutamate, alone or in association with additional substances, for the preparation of a medicament for the treatment of hyperhomocysteinemia. Said additional substances preferably are folic acid and vitamin B₁₂ in the form of folinic acid, in particular calcium pentahydrate salt, and cobamamide, respectively.

It has particularly been found that, contrary to what stated in the scientific literature previously mentioned, the presence of arginine glutamate in a composition containing folic acid and vitamin B₁₂ allows these last two compounds to determine an additional reduction in the homocysteine plasmatic concentration if administered in quantities greater than the ones mentioned in literature.

This allows administering to the single patient, and with a better effectiveness, a daily dose, intended as total quantity of administered composition in a whole day, clearly greater than the one known in literature.

Therefore it is an additional object of the present invention the use of arginine glutamate, folic acid and vitamin B₁₂ for the production of a medicament for the treatment of hyperhomocysteinemia, wherein the treatment involves an administration of a daily dose from 0.1 to 25 mg of folinic acid, from 0.1 to 5 mg of cobamamide and from 0.1 to 5000 mg of arginine glutamate.

The folic acid, the vitamin B₁₂ and the arginine glutamate are compounds known in the literature and easily available on the market or they can be prepared according to known methods. In particular according to the present invention the folic acid is used in the form of folinic acid, in particular in the form of calcium pentahydrate salt, and the vitamin B₁₂ in the form of cobamamide, compounds which are easily available on the market or which can be prepared according to known methods as well.

In a variant of the present invention the medicament is formulated as a kit the components thereof are kept separated; in particular the folinic acid and the cobamamide, optionally in presence of usual excipients, are present in solid form and separated from the arginine glutamate which is present in solution, optionally together with usual excipients and solvents. In a particularly preferred embodiment the three components are contained in a small bottle wherein the cap contains the folinic acid and the cobamamide optionally together with other excipients, whereas the small bottle contains the arginine glutamate in solution, optionally together with other excipients.

In another variant of the present invention the three components are kept together in the form of powder inside a suitable capsule, tablet, paper or additional forms of pharmaceutical presentation, with no degradation forms or decreasing in the activity of the composition and/or the single components taking place.

It is also an object of the present invention a pharmaceutical composition comprising folic acid, vitamin B₁₂, arginine glutamate and pharmaceutically tolerable additives to be used in the treatment of hyperhomocysteinemia, being excluded the composition wherein said arginine glutamate is present in quantities of 1000-mg. In said composition the folinic acid, preferably in the form of calcium pentahydrate salt, is contained in quantities between 0.1 to 25 mg, the cobamamide in quantities between 0.1 to 5 mg and the arginine glutamate in a quantity between 0.1 to 5000 mg.

### Pharmaceutical formulations

### Example 1

According to the present invention the following pharmaceutical compositions have been prepared, the quantities in active principles thereof are shown.

Vitamin B₁₂ in the form of cobamamide 0.2 mg, folic acid in the form of folinic acid, calcium pentahydrate salt 0.8, 1.0 and 2.0 mg, respectively, and arginine glutamate 1000, 1200 and 1400 mg, respectively. Said compositions have been called Composition 1 (0.2/0.8/1000), Composition 2 (0.2/1.0/1200) and Composition 3 (0.2/2.0/1400).

The compositions of the following examples have been prepared with the active substances of the example 1.

### Example 2

The following compositions have been prepared containing: vitamin B₁₂ 0.8 mg, folic acid 0.8-4.0-8.0 mg, respectively, and arginine glutamate 2000, 4000 and 5000 mg, respectively. Said compositions have been called Composition 4 (0.8/0.8/2000), Composition 5 (0.8/4/4000) and Composition 6 (0.8/8/5000).

### Example 3

The following compositions have been prepared containing: vitamin B₁₂ 1 mg, folic acid 2.2-4.4-5-6 mg, respectively, and arginine glutamate 1000. Said compositions have been called Composition 7 (1/2.2/1000), 8 (1/4.4/1000) 9 (1/5/1000) and 10 (1/6/1000).

### Example 4

The following compositions have been prepared: vitamin B₁₂ 1.4 mg, folic acid 2.8-5.6-7.2 mg, respectively, arginine glutamate 1000, 2000 and 5000 mg, respectively. These compositions have been designated as Composition 11, (1.4/2.8/1000), 12 (1.4/5.6/2000) and 13 (1.4/7.2/5000).

### Example 5

The following compositions have been prepared: vitamin B₁₂ 3 mg and folic acid 7-14-21 mg, respectively, and arginine glutamate 1000, 2000 e 5000 mg, respectively. These compositions have been designated as Composition 14, (3/7/1000) 15 (3/14/2000) and 16 (3/21/5000).

### Example 6

The following compositions have been prepared: Vitamin B₁₂ 5 mg, folic acid mg 10-15-20-25, respectively, and arginine glutamate 1000, 2000, 4000 e 5000 mg, respectively. These compositions have been designated as Composition 17 (5/10/1000), 18 (5/15/2000), 19 (5/20/4000) and 20 (5/25/5000).

### Example 7

3 compositions have been prepared containing 1000-2000-5000 mg of arginine glutamate, respectively, which have been designated as compositions 21, 22 and 23.

### Pharmacological assessments

Assessments about the pharmacological effectiveness of the composition according to the present invention have been carried out according to the following study protocol.

### Number of patients

The experimentation has examined a total number of 60 patients assigned to one of the two treatment groups (30 patients to group A and 30 patients to group B).

### Inclusion criteria

- Patients of both sexes
- Age older than 18
- Homocysteinemia level >15 µmol/l

### Exclusion criteria

- Patients suffering from diseases interfering with the homocysteine dosage
- Patients in therapy with drugs interfering with the homocysteine metabolism (isoniazid, antiepileptic, methotrexate)
- Subjects with ascertained individual hypersensitivity towards the active principles and/of the excipients of the product
- Women during pregnancy or puerperium
- Patients suffering from diabetes mellitus

The patients have been assigned in random order to one of the following treatment groups:
Group A): composition containing folinic acid, calcium pentahydrate salt 5 mg, cobamamide 1 mg and arginine glutamate 1000 mg (composition 9) formulated as a 10-ml small bottle which can be drunk, to be taken twice a day for 30 days.
Group B): a composition containing 1000 mg of arginine glutamate (composition 21) formulated as a 10-ml small bottle which can be drunk, to be taken twice a day for 30 days.

### Randomization and delivery of drugs

The assignment of the patients to the single group has been carried out by means of a randomization list.

The drug packages have been delivered to the patients together with a closed envelope wherein the intake modes have been explained.

During the whole period of therapy the intake of drugs interfering with the homocysteine metabolism and listed in the exclusion criteria has not been allowed.

### Effectiveness assessment

The homocysteine ematic level has been the effectiveness parameter of the study.

The patients have been subjected to 2 ambulatory examinations in total and to 2 ematic takings (at time of enrolment and after 30 days of treatment); the second taking has been carried out within 24-72 hours from the last drug intake.

The homocysteine determination has been carried out on venous blood gathered in test tubes containing EDTA, on patients who have not eaten at least for 12 hours. The ematic sample has been centrifugated at 3000 rpm for 15 minutes and on the plasma the determination of the homocysteine levels has been carried out by means of a immunoenzimatic test (EIA).

The obtained data have been assessed statistically withe Student's t test and the significance is expressed as follows: P<0.05*; P<0.01**; P<0.001***

The pharmacological results of the study carried out according to the preceding scheme have underlined a reduction of 51% (p≤0.001) in the homocysteine plasmatic values in the patients treated with a previously shown composition. The administration of arginine glutamate only has caused a statistically significative lowering (p≤0.01) in the homocysteine plasmatic values valuable to 12%. Additional studies carried out with compositions of the examples from 1 to 7 have substantially confirmed the previously shown data.

## Claims

1. Use of folic acid, vitamin B₁₂ and arginine glutamate for the production of a medicament for the treatment of hyperhomocysteinemia.

2. Use according to claim 1, wherein folic acid is present in the form of folinic acid and/or salts thereof and vitamin B₁₂ in the form of cobamamide.

3. Use according to claim 2, wherein said folinic acid is present in the form of calcium pentahydrate salt in a quantity from 0.1 to 25 mg, said cobamamide in a quantity from 0.1 to 5 mg and said arginine glutamate in a quantity from 0.1 to 5000 mg.

4. Use according to claim 3, wherein said folinic acid is present in the form of calcium pentahydrate salt in a quantity of 5 mg, said cobamamide in a quantity of 1 mg and said arginine glutamate in a quantity of 1000 mg.

5. Use according to claim 4, wherein said medicament is formulated as a kit wherein said folinic acid and cobamamide are present in solid form and, optionally together with usual excipients, kept separated from said arginine glutamate which is present in solution, together with usual excipients.

6. Use according to claim 5, wherein in said medicament said folinic acid and cobamamide are contained into the cap of a small bottle and said arginine glutamate in said small bottle.

7. Use of arginine glutamate, alone or in association with other substances, for the preparation of a medicament for the treatment of hyperhomocysteinemia.

8. Use according to claim 7, wherein said other substances are folic acid and vitamin B₁₂.

9. Use according to claim 8, wherein said folic acid is present in the form of folinic acid and/or salts thereof and said vitamin B₁₂ is present in the form of cobamamide.

10. Use according to claim 9, wherein said folinic acid is present in the form of calcium pentahydrate salt.

11. Use according to claims from 7 to 10, wherein said treatment involves an administration of a daily dose from 0.1 to 25 mg of folinic acid, in the form of calcium pentahydrate salt from 0.1 to 5 mg of cobamamide and from 0.1 to 5000 mg of arginine glutamate.

12. Pharmaceutical composition comprising folic acid, vitamin B₁₂, arginine glutamate and pharmaceutically tolerable additives for using in the treatment of hyperhomocysteinemia, the composition wherein said arginine glutamate is present in quantity of 1000 mg being excluded.

13. Composition according to claim 12, wherein the folic acid is present in the form of folinic acid and/or salts thereof and the vitamin B₁₂ in the form of cobamamide.

14. Composition according to claim 13, wherein said folinic acid is present in the form of calcium pentahydrate salt in a quantity from 0.1 to 25 mg, said cobamamide in a quantity from 0.1 to 5 mg and said arginine glutamate in a quantity from 0.1 to 5000 mg.

15. Composition according to claim 14, wherein said medicament is formulated as kit wherein said folinic acid and cobamamide are present in solid form and, optionally together with usual excipients, they are kept separated from said arginine glutamate which is present in solution, together with usual excipients.

16. Composition according to claim 15, wherein said folinic acid and cobamamide are kept in the cap of a small bottle and said arginine glutamate in said small bottle.

## Patentansprüche

1. Verwendung von Folsäure, Vitamin B₁₂ und Argininglutamat zur Herstellung eines Medikaments zur Behandlung von Hyperhomocysteinämie.

2. Verwendung gemäß Anspruch 1, worin Folsäure in Form von Folinsäure und/oder Salzen davon und Vitamin B₁₂ in Form von Cobamamid vorliegen.

3. Verwendung gemäß Anspruch 2, worin die Folinsäure in Form von Calcium-Pentahydrat-Salz in einer Menge von 0,1 bis 25 mg, das Cobamamid in einer Menge von 0,1 bis 5 mg und das Argininglutamat in einer Menge von 0,1 bis 5000 mg vorliegen.

4. Verwendung gemäß Anspruch 3, worin die Folinsäure in Form von Calcium-Pentahydrat-Salz in einer Menge von 5 mg, das Cobamamid in einer Menge von 1 mg und das Argininglutamat in einer Menge von 1000 mg vorliegen.

5. Verwendung gemäß Anspruch 4, worin das Medikament als ein Kit formuliert ist, worin die Folinsäure und Cobamamid in fester Form und optional zusammen mit üblichen Arzneimittelträgern vorliegen, getrennt gehalten von dem Argininglutamat, das in Lösung vorliegt, zusammen mit üblichen Arzneimittelträgern.

6. Verwendung gemäß Anspruch 5, worin in dem Medikament die Folinsäure und Cobamamid in der Kappe einer kleinen Flasche und das Argininglutamat in der kleinen Flasche enthalten sind.

7. Verwendung von Argininglutamat, allein oder in Verbindung mit anderen Substanzen, zur Herstellung eines Medikaments zur Behandlung von Hyperhomocysteinämie.

8. Verwendung gemäß Anspruch 7, worin die anderen Substanzen Folsäure und Vitamin B₁₂ sind.

9. Verwendung gemäß Anspruch 8, worin die Folsäure in Form von Folinsäure und/oder Salzen davon und das Vitamin B₁₂ in Form von Cobamamid vorliegen.

10. Verwendung gemäß Anspruch 9, worin die Folinsäure in Form von Calcium-Pentahydrat-Salz vorliegt.

11. Verwendung gemäß den Ansprüchen 7 bis 10, worin die Behandlung eine Verabreichung einer täglichen Dosis von 0,1 bis 25 mg Folinsäure in Form von Calcium-Pentahydrat-Salz, von 0,1 bis 5 mg Cobamamid und von 0,1, bis 5000 mg Argininglutamat umfasst.

12. Pharmazeutische Zusammensetzung, umfassend Folsäure, Vitamin B₁₂, Argininglutamat und pharmazeutisch annehmbare Zusätze zur Verwendung bei der Behandlung von Hyperhomocysteinämie, wobei die Zusammensetzung, worin das Argininglutamat in einer Menge von 1000 mg vorhanden ist, ausgeschlossen ist.

13. Zusammensetzung gemäß Anspruch 12, worin die Folsäure in Form von Folinsäure und/oder Salzen davon und das Vitamin B₁₂ in Form von Cobamamid vorhanden sind.

14. Zusammensetzung gemäß Anspruch 13, worin die Folinsäure in Form von Calcium-Pentahydrat-Salz in einer Menge von 0,1 bis 25 mg, das Cobamamid in einer Menge von 0,1 bis 5 mg und das Argininglutamat in einer Menge von 0,1 bis 5000 mg vorhanden ist.

15. Zusammensetzung gemäß Anspruch 14, worin das Medikament als Kit formuliert ist, worin die Folinsäure und Cobamamid in fester Form und optional zusammen mit üblichen Arzneimittelträgern vorhanden sind, sie von dem Argininglutamat getrennt gehalten sind, das in Lösung vorhanden ist, zusammen mit üblichen Arzneimittelträgern.

16. Zusammensetzung gemäß Anspruch 15, worin die Folinsäure und Cobamamid in der Kappe einer kleinen Flasche und das Argininglutamat in der kleinen Flasche gehalten sind.

## Revendications

1. Utilisation de l'acide folique, de la vitamine B₁₂ et du glutamate d'arginine pour la production d'un médicament destiné au traitement de l'hyperhomocystéinémie.

2. Utilisation selon la revendication 1, dans laquelle l'acide folique est présent sous forme d'acide folinique et/ou de ses sels et de la vitamine B₁₂ sous forme de cobamamide.

3. Utilisation selon la revendication 2, dans laquelle ledit acide folinique est présent sous forme de sel de pentahydrate de calcium dans une quantité de 0,1 à 25 mg, ledit cobamamide est dans une quantité de 0,1 à 5 mg et ledit glutamate d'arginine dans une quantité de 0,1 à 5000 mg.

4. Utilisation selon la revendication 3, dans laquelle ledit acide folinique est présent sous forme de sel de pentahydrate de calcium dans une quantité de 5 mg, ledit cobamamide dans une quantité de 1 mg et ledit glutamate d'arginine dans une quantité de 1000 mg.

5. Utilisation selon la revendication 4, dans laquelle ledit médicament est formulé sous forme de kit dans lequel ledit acide folinique et le cobamamide sont présents dans une forme solide et, facultativement, ensemble avec les excipients habituels, maintenus séparés du glutamate d'arginine qui est présent en solution conjointement avec les excipients habituels.

6. Utilisation selon la revendication 5, dans laquelle dans ledit médicament, ledit acide folinique et le cobamamide sont contenus dans le capuchon d'un petit flacon et ledit glutamate d'arginine dans ledit petit flacon.

7. Utilisation de glutamate d'arginine, seul ou en association avec d'autres substances, pour la préparation d'un médicament pour le traitement d'hyperhomocystéinémie.

8. Utilisation selon la revendication 7, dans laquelle lesdites autres substances sont l'acide folique et la vitamine B₁₂.

9. Utilisation selon la revendication 8, dans lequel ledit acide folique est présent sous forme d'acide folinique et/ou de ses sels, et ladite vitamine B₁₂ est présente sous forme de cobamamide.

10. Utilisation selon la revendication 9, dans laquelle ledit acide folinique est présent sous forme de sel de pentahydrate de calcium.

11. Utilisation selon les revendications 7 à 10, dans laquelle ledit traitement applique une administration d'une dose quotidienne de 0,1 à 25 mg d'acide folinique, sous forme de sel de pentahydrate de calcium de 0,1 à 5 mg de cobamamide et de 0,1 à 5000 mg de glutamate d'arginine.

12. Composition pharmaceutique comprenant l'acide folique, la vitamine B₁₂, le glutamate d'arginine et des additifs tolérables pharmaceutiquement pour l'utilisation dans le traitement de l'hyperhomocystéinémie, la composition dans laquelle ledit glutamate d'arginine est présent dans une quantité de 1000 mg étant exclue.

13. Composition selon la revendication 12, dans laquelle l'acide folique est présent sous forme d'acide folinique et/ou de ses sels, et la vitamine B₁₂ sous forme de cobamamide.

14. Composition selon la revendication 13, dans laquelle ledit acide folinique est présent sous forme de sel de pentahydrate de calcium dans une quantité de 0,1 à 25 mg, ledit cobamamide dans une quantité de 0,1 à 5 mg et ledit glutamate d'arginine dans une quantité de 0,1 à 5000 mg.

15. Composition selon la revendication 14, dans laquelle ledit médicament est formulé sous forme de kit dans lequel ledit acide folinique et le cobamamide sont présents sous forme solide et, facultativement, conjointement avec les excipients habituels, ils sont maintenus séparés dudit glutamate d'arginine qui est présent en solution, conjointement avec les excipients habituels.

16. Composition selon la revendication 15, dans laquelle ledit acide folinique et le cobamamide sont maintenus dans le capuchon d'un petit flacon et ledit glutamate d'arginine dans ledit petit flacon.
